# FASCICULE DE BREVET EUROPEEN

(11) **EP 2 877 590 B1**
(45) Date de publication et mention de la délivrance du brevet: **18.05.2016**
(21) Numéro de dépôt: 13756524.8
(22) Date de dépôt: 24.06.2013
(51) Int. Cl.: C12Q 1/04, G01N 33/68

(54) **MÉTHODE DE DIAGNOSTIC IN VITRO D'UNE INFECTION FONGIQUE INVASIVE PAR SPECTROMÉTRIE DE MASSE MALDI-TOF**
VERFAHREN ZUR IN-VITRO-DIAGNOSE EINER INVASIVEN PILZINFEKTION MITTELS MALDI-TOF-MASSENSPEKTROMETRIE
METHOD FOR THE IN VITRO DIAGNOSIS OF AN INVASIVE FUNGAL INFECTION BY MALDI-TOF MASS SPECTROMETRY

(30) Priorité: 26.06.2012 FR 1201796
(43) Date de publication de la demande: 03.06.2015
(73) Titulaire: Centre Hospitalier Regional Universitaire de Lille, 59000 Lille (FR); Université de Droit et Santé de Lille, 59 800 Lille (FR); Université Sciences Technologies Lille, 59655 Villeneuve d'Ascq Cedex (FR); Centre National de la Recherche Scientifique (CNRS), 75794 Paris Cedex 16 (FR)
(72) Inventeur: POULAIN, Daniel, F-59 242 Templeuve (FR); SENDID, Boualem, F-59120 Loos (FR); GUERARDEL, Yann, F-59790 Ronchin (FR); FRANCOIS, Nadine, F-59800 Lille (FR)
(74) Mandataire: Schwalek, Valérie
(86) Numéro de dépôt international: PCT/FR2013/000158
(87) Numéro de publication internationale: WO 2014/001658

(56) Documents cités:
- WO-A2-2005/111627
- US-A1- 2010 003 699
- R WHISTLER ET AL: "Chromatographic separation of sugars on charcoal", JOURNAL OF THE AMERICAN CHEMICAL SOCIETY, vol. 72, no. 2, 1 janvier 1950 (1950-01-01), pages 677-679, XP55060186, ISSN: 0002-7863 cité dans la demande
- FIEDLER G M ET AL: "Standardized peptidome profiling of human urine by magnetic bead separation and matrix-assisted laser desorption/ionization time-of-flight mass spectrometry", CLINICAL CHEMISTRY, vol. 53, no. 3, 1 mars 2007 (2007-03-01), pages 421-428, XP002505366, ISSN: 0009-9147, DOI: 10.1373/CLINCHEM.2006.077834
- HA M-Y ET AL: "Development of a matrix-prespotted plate for enhancing the reproducibility of serum glycan analysis by MALDI-TOF-MS", MASS SPECTROMETRY LETTERS, vol. 2, no. 3, 15 septembre 2011 (2011-09-15), pages 61-64, XP055059988, ISSN: 2233-4203, DOI: 10.5478/MSL.2011.2.3.061
- QIAN J ET AL: "MALDI-TOF mass signatures for differentiation of yeast species, strain grouping and monitoring of morphogenesis markers", ANALYTICAL AND BIOANALYTICAL CHEMISTRY, vol. 392, no. 3, 9 août 2008 (2008-08-09), pages 439-449, XP019621641, ISSN: 1618-2650
- HO Y-P ET AL: "Advances in mass spectrometry for the identification of pathogens", MASS SPECTROMETRY REVIEWS, vol. 30, no. 6, 9 mai 2011 (2011-05-09), pages 1203-1224, XP055059785, ISSN: 0277-7037, DOI: 10.1002/mas.20320

## Description

La présente invention concerne une méthode de diagnostic *in vitro* d'une infection fongique invasive par spectrométrie de masse MALDI-TOF. La méthode de l'invention permet également, la quantification d'un composé d'intérêt contenu dans l'échantillon et utilisé comme marqueur pour le diagnostic d'une infection fongique invasive. Les levures comme *C*. *albicans* sont souvent présentes dans les muqueuses des individus sains chez lesquels elles n'entraînent habituellement aucune maladie ou symptôme particulier. Lorsque l'organisme est affaibli les levures prolifèrent et passent dans le système sanguin : on parle alors d'infection invasive ou systémique. Dans le cas de *C*. *albicans,* 40% des candidémies (fongémies causées par *C*. *albicans)* sont fatales chez l'Homme. Les maladies fongiques invasives (MFI) ou infections fongiques invasives sont des affections fréquentes et graves en milieu hospitalier. Malgré des thérapeutiques efficaces dont le coût constitue une charge de plus en plus difficile à supporter par les hôpitaux, la morbimortalité des MFI ne décroit pas. Les méthodes mycologiques conventionnelles (isolement/identification) sont souvent en défaut car l'accès, sans risque pour le patient, à des sites d'infection localisés afin d'y prélever le champignon est souvent impossible. Par ailleurs, les hémocultures sont négatives dans près de la moitié des cas de MFI. Les méthodes dites de biologie moléculaire (PCR) ne résolvent pas ces problèmes. De plus, il est enfin établi que la précocité de mise en oeuvre du traitement antifongique -sur la base d'un diagnostic- conditionne la survie du patient.Parmi les méthodes diagnostiques complémentaires ou alternatives à la mycologie conventionnelle, l'utilité des méthodes de détection de glycanes circulants (c'est-à-dire les glycanes fongiques contenus dans le sérum des patients infectés) dans les sérums de patients est maintenant reconnue par les cliniciens. Ces glycanes proviennent de la paroi des micromycètes ou de ses précurseurs et peuvent être détectés par des tests immunologiques commerciaux. Ainsi, le kit Platelia® Candida Antigen permet de détecter les mannanes et les galactomannanes provenant, respectivement, de Candida et d'Aspergillus. Des kits de tests biochimiques, tels que celui commercialisé sous la marque Fungitell®, par exemple, permettent de détecter les glucanes communs aux Candida et aux Aspergillus.

Chaque «kit» des deux types précités comprend de nombreux réactifs et standards internes différents. La nécessité de réaliser des courbes d'étalonnage à chaque série amène à une consommation accrue de réactifs normalement destinés à tester des sérums. Cette nécessaire standardisation peut parfois amener à remettre des examens pour des raisons économiques (faute d'une quantité suffisante de réactifs). Il est, par exemple, difficile en ce qui concerne le Fungitell® de justifier l'utilisation de 9 puits pour standardisation et la monopolisation d'un agent technique une demi-journée pour tester un sérum. Ces tests nécessitent également l'accès à des automates programmés spécifiquement et présentant des interfaces avec le système informatique ce qui complique encore leur pratique unitaire en urgence alors qu'ils devraient être à même de répondre dans les plus brefs délais à leur prescription.

Tous ces tests sont donc particulièrement onéreux en temps et/ou en réactifs. De plus, il existe toujours un certain nombre de faux positifs qui perturbent le diagnostic. Ceci est particulièrement vrai pour les dosages de glucane(s) par voie biochimique qui sont perturbés par la présence d'hémoglobine (sérums hémolysés lors de leur recueil) ou de perturbations métaboliques fréquentes chez les patients à risque de MFI telles qu'une hypertriglycéridémie, la présence de bilirubine ou d'une hyperprotéinémie. Enfin la présence de faux positifs chez les patients hospitalisés en réanimation et pluri-infectés par d'autres germes que les champignons a été rapportée à plusieurs reprises. La nature des mécanismes conduisant à cette fausse positivité de la détection des β-D-glucanes reste inconnue faute de caractérisation moléculaire des produits qui circulent dans les sérums de ces patients. Du fait de la présence de ces faux positifs, certains patients qui ne nécessiteraient aucun traitement se voient administrer une antibiothérapie antifongique qui prolonge leur séjour à l'hôpital. Dans les cas des faux négatifs qui existent aussi, on considère qu'il n'y a pas d'infection fongique mais une infection bactérienne ; le patient se voit donc administrer une antibiothérapie antibactérienne qui lui est néfaste et peut lui être fatale.

Par ailleurs, le kit Fungitell® utilise du sang de limule, un animal qui commence à se raréfier. En effet, à l'heure actuelle il est impossible d'élever les limules. Les limules sont donc capturés dans leur milieu naturel. On leur prélève une fraction de leur sang avant de les relâcher. Quinze pourcents des limules relâchés ne survivent pas.

Un but de la présente invention est de proposer une nouvelle méthode *in vitro* de diagnostic d'une infection fongique invasive.

Un autre but est de proposer une méthode *in vitro* telle que précitée qui soit plus fiable, en particulier, une méthode qui permet d'éviter les faux négatifs et/ou faux positifs décrits précédemment.

La présente invention concerne une méthode de diagnostic *in vitro* d'une infection fongique invasive causée par un micro-organisme fongique pathogène, selon la revendication 1.

Chacun des documents WO 2005/111627 A2, US 2010/003699 A1 et MASS SPECTROMETRY LETTERS, vol. 2, no. 3, le 15 septembre 2011, pages 61-64, XP055059988, ISSN 2233-4203 décrit un procédé pour diagnostiquer une maladie, et surtout le cancer, en déterminant la présence/absence des biomarqueurs glycanes dans un échantillon de liquide biologique par moyen de la spectrométrie de masse de type MALDI-TOF. Mais il est à noter que les biomarqueurs détectés dans l'art antérieur proviennent du sujet de l'examen et sont alors endogènes.

Un mérite des inventeurs est donc d'avoir pu montrer qu'il était possible par spectrométrie de masse de type MALDI-TOF de détecter, dans un liquide biologique complexe, la présence d'un composé choisi parmi les sucres, en particulier les polysaccharides, les oligosaccharides et les monosaccharides qui provient du micro-organisme pathogène et non du mammifère hôte. Un autre mérite des inventeurs est d'avoir montré que ce composé d'intérêt s'avère être un marqueur pour le diagnostic des infections fongiques invasives. La présence du composé d'intérêt (marqueur) correspond à un pic visible sur le spectre obtenu par spectrométrie de masse de type MALDI-TOF. Il n'était pas évident en effet qu'un composé tel que précité provenant d'un micro-organisme fongique pathogène puisse être détecté par spectrométrie de masse de type MALDI-TOF dans un liquide biologique. Il était encore moins évident que ce composé d'intérêt puisse indiquer une infection fongique invasive. En effet, on pouvait penser que la quantité du composé d'intérêt serait trop faible pour être détectée ou que le signal correspondant à ce composé d'intérêt serait masqué parmi les milliers de signaux pouvant être observés dans un milieu biologique, notamment sérique. De plus, les sucres (polysaccharides, oligosaccharides et monosaccharides) forment dans les liquides biologiques des complexes avec les lipides, protéines et sels et sont donc difficilement détectables.

Le ou les pics significatifs et correspondant à un ou à des composés d'intérêt permettant de diagnostiquer une infection fongique invasive causée par un micro-organisme donné sont préalablement mis en évidence en reproduisant, par exemple, le protocole expérimental décrit dans la présente demande avec des échantillons de liquide biologique provenant de mammifères infectés par le ou les micro-organismes fongique(s) pathogène(s) donné.

La méthode de l'invention permet de détecter des composés d'intérêt tels que précités sans nécessiter de les marquer.

Il est possible, selon la méthode de l'invention de détecter par spectrométrie de masse de type MALDI-TOF la présence simultanée de plusieurs composés d'intérêt dont l'un au moins fait partie des polysaccharides, oligosaccharides ou monosaccharides. Tous les composés d'intérêt peuvent également, selon l'invention être des sucres tels que précités. Avantageusement, pour extraire ledit composé d'intérêt, on dissocie lesdits complexes contenus dans ledit échantillon de liquide biologique par précipitation/coagulation de la majorité desdites protéines et/ou desdits lipides ;
- on centrifuge ledit échantillon et on sépare le surnageant de la fraction solide ;
- on récupère le surnageant ;
- on sépare ledit composé d'intérêt contenu dans le surnageant, des éventuelles protéines résiduelles et des éventuels lipides résiduels, notamment, par chromatographie de phase inverse et on sépare ledit composé d'intérêt desdits sels, notamment en réalisant une chromatographie d'absorption.

Selon l'invention, il est possible de séparer d'abord le composé d'intérêt des sels puis de le séparer des éventuelles protéines résiduelles et/ou lipides résiduels ou d'effectuer ces deux étapes dans l'ordre inverse.

Avantageusement, pour dissocier lesdits complexes, on ajoute un agent complexant audit échantillon, notamment une base, en particulier de l'EDTA, et on chauffe ledit mélange obtenu, jusqu'à ébullition et en particulier à une température supérieure ou égale à 100°C et inférieure ou égale à 140°C et notamment, sensiblement égale à 120°C.

La durée du chauffage peut être ajustée par l'Homme du Métier en fonction de la taille de l'échantillon, de la technique utilisée pour le chauffage et du récipient contenant l'échantillon. La totalité du volume de l'échantillon doit être portée à ébullition. La durée de chauffage est adaptée pour ne pas dégrader l'échantillon, en particulier les sucres qu'il contient. A titre d'exemple, la durée de chauffage est comprise entre 3 minutes et 7 minutes.

La méthode de l'invention est particulièrement robuste et d'un coût très faible en matière de réactifs ; en termes de comptabilité analytique le temps technicien n'est pas supérieur à celui lié à l'utilisation des kits existants. L'agent complexant, par exemple l'EDTA associé au chauffage permettent de dissocier les complexes existant dans le liquide biologique brut. L'agent complexant permet notamment de libérer les sucres de lectines dépendantes de cation divalents

Selon un mode de réalisation particulier, on effectue d'abord la chromatographie en phase inverse puis la chromatographie d'absorption.

Avantageusement, on utilise une colonne comportant une phase solide hydrophobe pour la chromatographie inverse et une colonne contenant du charbon actif pour la chromatographie d'absorption. La colonne hydrophobe peut être une colonne remplie de tout support solide sur lesquels sont greffés, par exemple, des groupements de type octadecyl (C18). Le support peut être un polymère ou de la silice poreuse. La taille et le volume de ces colonnes seront variables en fonction du volume de sérum à traiter.

La colonne contenant du charbon actif contient un mélange en poids égal de charbon actif et de célite® (terre de diatomée), ce qui permet de récupérer dans l'éluât les mono et disaccharides, les autres saccharides étant retenus dans la colonne.

La taille et la masse molaire du composé d'intérêt ne sont pas limitées selon l'invention. Le composé d'intérêt peut, par exemple, présenter un rapport m/z inférieur à 1000. Le composé d'intérêt peut être un disaccharide, en particulier un dimère d'hexose correspondant à un signal à m/z= 365. Ce composé d'intérêt qui est un marqueur d'une infection fongique invasive peut être, par exemple, le tréhalose.

Avantageusement, après ajout dudit agent complexant, on ajoute au moins une enzyme capable de dégrader les polysaccharides et/ou oligosaccharides et/ou on effectue un traitement chimique afin de couper les chaînes desdits polysaccharides et/ou oligosaccharides, moyennant quoi, on augmente potentiellement la quantité dudit composé d'intérêt.

L'utilisation d'endo-mannosidases permet de libérer des oligomannosides libres afin d'augmenter l'intensité de leurs signaux en spectrométrie de masse (MALDI-TOF). Un traitement chimique, acétolyse, par exemple, peut également être envisagé dans ce cas. Il est également possible d'agir de même sur les galactomannanes, les glucanes, voire la chitine (trois composants majoritaires au sein de la paroi fongique) de façon à libérer des courtes chaînes à partir de ces polymères.

Ainsi, une augmentation de l'intensité du signal d'un tri saccharide après mannosidase permet d'attribuer le signal à des mannanes circulants.

Cette démarche analytique peut se compléter de l'action d'exo-glycosidases pour lesquelles les conclusions seront basées à l'inverse sur la disparition du signal. On peut ainsi, parvenir à déterminer le pathogène impliqué en fonction du type d'oligosaccharides présents dans l'échantillon de liquide biologique.

Avantageusement, ledit composé d'intérêt est choisi parmi les monosaccharides et oligosaccharides suivants : [N-acétyl-glucosamine β-(1,4) N-acétyl-glucosamine]n ou n est un entier supérieur ou égal à 1 et inférieur ou égal à 10, les oligosaccharides comprenant de 2 à 10 monosaccharides, notamment les oligomères d'hexose, en particulier les dimères d'hexose, les glucanes, notamment les glucanes du type [glucose β-(1,3) glucose]n, [glucose β-(1,6) glucose]n où n est un entier supérieur ou égal à 1 et inférieur ou égal à 10; les mannanes, notamment les mannanes du type [mannose α-(1,2) mannose]ₙ, [mannose α-(1,3) mannose]ₙ, [mannose α-(1,6) mannose]ₙ, [mannose β-(1,2) mannose]ₙ où n est un entier supérieur ou égal à 1 et inférieur ou égal à 10, les galactomannanes, les galactanes, les arabinogalactanes et les glucuronoxylomannanes.

Selon un mode de mise en oeuvre particulier de la méthode de l'invention, le composé d'intérêt est un composé produit par le micro-organisme fongique pathogène et plus particulièrement le tréhalose.

Le composé d'intérêt servant de marqueur peut être un dimère d'hexose, en particulier le tréhalose (m/z=365). Un tel type de composé d'intérêt s'est avéré être un bon marqueur d'une infection par un pathogène, en particulier d'une infection par *Candida albicans.*

Le micro-organisme fongique pathogène peut être choisi parmi les micro-organismes fongiques pathogènes capables de produire du tréhalose sous l'action du système immunitaire dudit mammifère, *Candida spp., en particulier Candida albicans, Aspergillus spp., Fusarium spp., Trichosporon spp., Saccharomyces cerevisiae, Acremonium spp., Coccidioides immitis, Histoplasma capsulatum, Sporothrix schenckii* et *Pneumocystis jirovecii.*

Avantageusement, le micro-organisme fongique pathogène est choisi parmi les micro-organismes fongiques pathogènes précités qui sous l'action du système immunitaire des mammifères, produisent du tréhalose.

Avantageusement, on ajoute audit échantillon une quantité connue d'au moins un composé étalon, moyennant quoi, en comparant l'intensité du signal obtenu pour ledit composé d'intérêt donné avec le signal obtenu pour ledit composé étalon, on peut déterminer la quantité dudit composé d'intérêt donné contenu dans ledit échantillon. Le composé étalon peut être ajouté à l'échantillon, avant traitement de celui-ci. Il est possible, selon l'invention, d'utiliser en tant que composé étalon des composés présentant des valeurs m/z proches de celle des oligosaccharides à analyser. On considère que des valeurs m/z appartenant à un intervalle [m/z du composé d'intérêt -5 ; m/z du composé d'intérêt +5] ou égales aux bornes de cet intervalle sont proches de la valeur m/z du composé d'intérêt.

Avantageusement, le composé étalon présente un comportement chromatographique sur les colonnes utilisées identique à ceux du composé à détecter. Il peut dans ce cas être ajouté après l'étape de chauffage et avant le passage sur les deux colonnes. Le composé étalon ne doit pas être un composé présent dans les liquides biologiques, qu'ils soient sains ou pathologiques. Le composé étalon ne doit pas être susceptible d'être identifié comme issu de contamination microbienne. On utilisera notamment avantageusement à ces fins du des disaccharides ou des tri saccharides, éventuellement marqués par un radio-isotope. Le tréhalose deutéré (MW 2H tréhalose=356+Na=379) peut, par exemple, être utilisé comme composé étalon.

Selon un autre mode de mise en oeuvre, on détermine le rapport de l'intensité du signal correspondant au composé d'intérêt donné sur l'intensité d'un signal endogène ubiquitaire, ledit signal endogène étant préalablement identifié et éventuellement spécifique du type de liquide biologique dont provient l'échantillon, moyennant quoi on peut quantifier ledit composé d'intérêt donné contenu dans ledit échantillon.

C'est en effet un autre mérite des inventeurs que d'avoir identifié un signal constant (dit signal ubiquitaire) présent dans tout échantillon de sérum qui peut servir pour la quantification du composé d'intérêt dans ce type de liquide biologique. L'Homme du Métier peut facilement déterminer un signal ubiquitaire en réalisant l'analyse MALDI-TOF de plusieurs échantillons (de préférence non infectés ou non suspectés comme tels) d'un type de liquide biologique donné. Le signal ubiquitaire correspond à un signal constant retrouvé pour tous les échantillons de ce type de fluide. L'intensité du signal telle que précitée correspond à la hauteur du pic apparaissant sur le spectrogramme obtenu par analyse MALDI-TOF.

Ainsi, selon l'invention, le liquide biologique peut être choisi parmi le sang veineux et artériel, le sérum, les liquides des muqueuses génitales, les liquides des voies aériennes, notamment, le liquide provenant de lavage broncho-alvéolaire, d'aspiration bronchique, d'aspiration trachéale, d'expectoration, les crachats, les liquides ayant servi à collecter des fragments de peau et des phanères, les liquides d'épanchement, les liquides des cavités closes, notamment, le liquide céphalo-rachidien, les liquides du système digestif ou urinaire et les liquides obtenus à partir de broyat de fragment de biopsie.

La présente invention concerne également un kit permettant la mise en oeuvre du procédé selon la revendication 14.

Le garnissage peut être préparé en mélangeant une quantité égale de charbon actif commercial et de Célite® (terre de diatomée) selon un principe bien connu de l'homme de métier et décrit dans la publication suivante : Whistler and Durso, Journal of American Chemical Society, 1950.

La phase solide hydrophobe se présente sous forme divisée et comporte un matériau magnétique, moyennant quoi il est possible de retirer la phase solide à l'aide d'un aimant après contact avec ledit liquide biologique. De tels matériaux sont connus du document CLINICAL CHEMISTRY, vol. 53, no. 3, le 1 mars 2007, pages 421-428, XP002505366, ISSN 0009-9147. Par exemple, la phase solide hydrophobe peut se présenter sous la forme d'une pluralité de billes contenant un matériau ferreux ou magnétique et recouvertes d'un matériau hydrophobe. On met en contact la phase solide hydrophobe sous forme divisée avec l'échantillon de liquide biologique. On mélange éventuellement. Les billes offrent une grande surface de contact. Après un certain temps de contact, on retire la phase solide hydrophobe avec un aimant, en plongeant, par exemple ce dernier dans le mélange liquide biologique-billes de phase solide hydrophobe. Les billes se collent à l'aimant ce qui permet de facilement séparer le liquide biologique traité.

Selon un autre mode de réalisation, le kit comprend, en outre, une quantité donnée d'un composé étalon choisi parmi les monosaccharides, les disaccharides et les tri saccharides, éventuellement marqués par un isotope radioactif, notamment le tréhalose deutéré

Avantageusement, le kit peut comprendre en outre, une quantité donnée d'une enzyme apte à réagir avec les complexes formés par les polysaccarides et/ou les oligosaccharides et/ou monosaccharides contenus dans un liquide biologique.

Le support comportant une phase solide hydrophobe permettant la mise en oeuvre d'une chromatographie en phase inverse précité peut être une plaque, un puits, une colonne, un tube, une plaque comportant une pluralité de puits.

Le récipient contenant le garnissage de charbon actif solide peut être un puits, une colonne, un tube, une plaque comportant une pluralité de puits, indépendamment du choix du support précité.

Avantageusement, le kit selon l'invention comporte en outre une plaque MALDI.

La présente invention concerne également l'utilisation d'un composé d'intérêt choisi parmi les polysaccharides, les oligosaccharides et les monosaccharides, en particulier le tréhalose pour le diagnostic *in vitro* d'une infection fongique invasive par spectrométrie de masse de type MALDI-TOF.

La présente invention, ses caractéristiques, particularités et divers avantages qu'elle procure apparaitront plus clairement à la lecture de la description qui suit et qui fait référence à un mode particulier de mise en oeuvre, présenté à titre d'exemple non limitatif et faisant référence aux dessins annexés parmi lesquels :

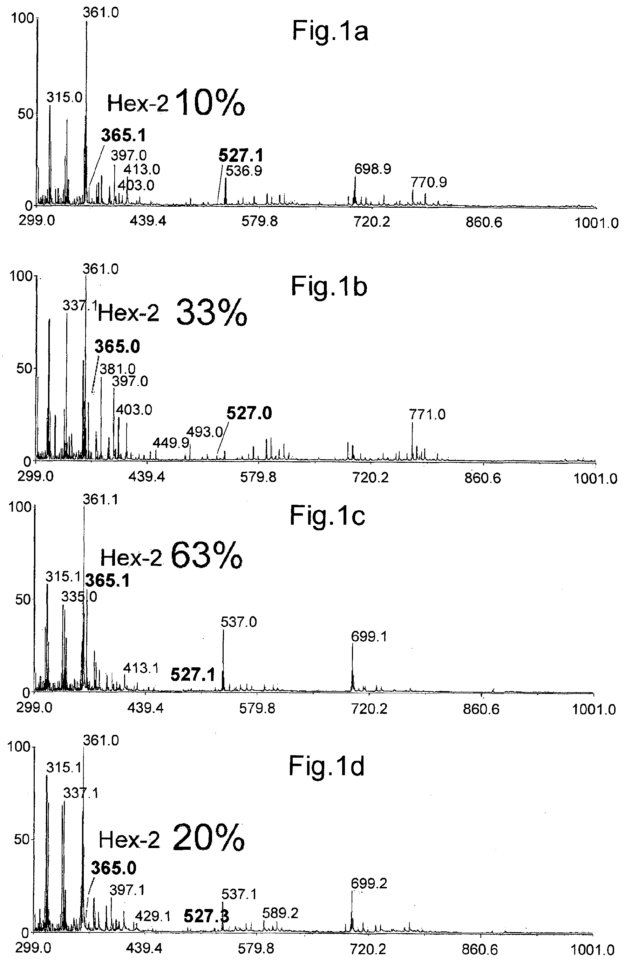

- Les Fig. 1a à 1d représentent, respectivement, les spectres de masse (MALDI-TOF) obtenus selon la méthode de l'invention par analyse des plaques MALDI préparées à partir des sérums G25, G32, G42 et G49 répertoriés dans le tableau I, un composé présent dans l'échantillon de sérum est représenté sur le spectre par un pic (ou signal), l'axe des abscisses du spectre indique le rapport m/z (en unité de masse m.u.) du composé représenté par le pic, l'axe des ordonnées indique l'intensité du signal (hauteur du pic) qui correspond à la quantité du composé représenté qui est contenue dans l'échantillon, la valeur 100% étant attribuée à un signal ubiquitaire endogène à tous les sérums ; et

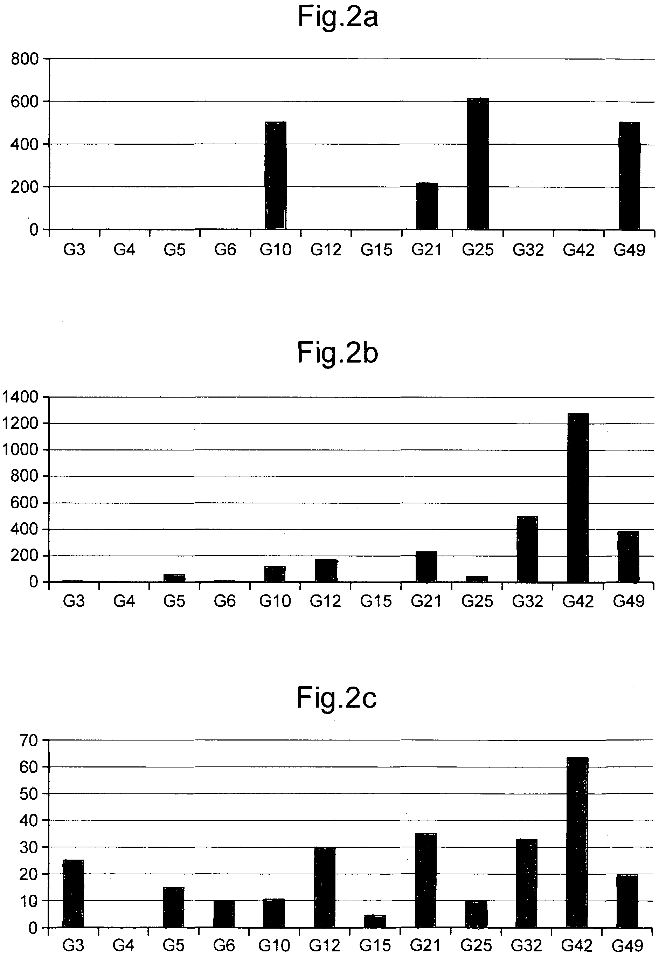

- La Fig. 2a représente sur l'axe vertical la quantité de mannanes (en ng/mL) contenus dans les échantillons de sérums identifiés dans le tableau I et qui sont reportés en abscisse, ladite quantité étant obtenue par utilisation du kit Platélia ® Candida antigen ;

- la Fig. 2b représente la quantité de glucanes (en ng/mL) contenus dans les échantillons de sérums du tableau I, la quantité de glucanes est reportée sur l'axe des ordonnées tandis que le numéro de sérum est indiqué en abscisse, ladite quantité est mesurée par utilisation du kit Fungitell®; et
- La Fig. 2c représente la quantité d'oligomères d'hexose (oligomère formé de deux hexoses plus amplement explicité ci-dessous) contenue dans chacun des échantillon du tableau I, la quantité d'oligomère précité en % du signal ubiquitaire est représentée en ordonnée, les groupes sont indiqués en abscisse, ladite quantité est obtenue par utilisation du procédé de l'invention et correspond au rapport de la hauteur du pic obtenu pour l'oligomère précité sur la hauteur du pic obtenu pour un signal ubiquitaire x100 ;

### Définitions

Selon l'invention, le mammifère n'est pas limité ; il peut s'agir de l'Homme ou de tout autre mammifère. La présente invention trouve donc une application en médecine humaine et en médecine vétérinaire.

Selon l'invention, les oligosaccharides et les polysaccharides sont définis comme étant des polymères de monosaccharides reliés entre eux par des liaisons glycosidiques. Les oligosaccharides sont constitués de 2 à 10 monosaccharides et les polysaccharides de plus de 10 monosaccharides.

Un spectromètre de type MALDI-TOF est un spectromètre de masse couplant une source d'ionisation laser assistée par une matrice (MALDI, Matrix-Assisted Laser Desorption/Ionisation) et un analyseur à temps de vol (TOF, time-of-flight mass spectrometry). Ce type d'appareil est actuellement assez répandu, y compris dans le milieu hospitalier.

Selon l'invention, on définit la présence du composé d'intérêt par la présence d'un pic visible sur le spectre de masse obtenu par spectrométrie MALDI-TOF. L'Homme du Métier est à même de reconnaître sur un tel spectre la présence d'un pic visible et donc significatif. En particulier, un pic significatif peut correspondre, selon l'invention, à un pic d'intensité supérieure ou égale à 1%, avantageusement supérieure ou égale à 3% et plus avantageusement au moins sensiblement égale à 5% par rapport à l'intensité d'un pic de référence.

Selon l'invention, les termes « infection fongique invasive » regroupent les infections qui provoquent la présence de levures (champignons) dans le sang périphérique du mammifère et les infections qui engendrent l'atteinte du sang et d'au moins un tissu. Dans le cas d'une infection par *C*. *albicans,* par exemple, dans laquelle il peut y avoir des atteintes hépatiques et rénales qui proviennent d'une dissémination hématogène et extravasculaire des levures, les termes « infection fongique invasive » regroupent la candidémie et la candidose invasive.

Selon l'invention, on définit un composé provenant d'un micro-organisme fongique pathogène comme étant tout résidu de micro-organisme fongique pathogène présent dans les liquides biologiques de l'hôte, spontanément libéré ou issu de la dépolymérisation/dégradation -par le micro-organisme lui-même ou par l'action des enzymes de l'hôte. Ainsi, le composé d'intérêt peut provenir de polysaccharides ou de copules glycaniques de glycoprotéines ou de glycolipides provenant du micro-organisme fongique pathogène. Le composé d'intérêt peut provenir de la paroi du micro-organisme fongique pathogène, d'un produit de dégradation de ce dernier, d'un composé secrété/excrété par ce dernier ou exprimé par ce dernier et libéré lors de la destruction dudit micro-organisme par les cellules de l'hôte. Il peut s'agir d'un composé qui provient d'une interaction entre le système immunitaire du mammifère infecté et ledit micro-organisme fongique pathogène.

Par «micro-organisme fongique pathogène» on entend les champignons (levures) dont l'équipement génétique permet de construire des séquences polysaccharidiques ou oligosaccharidiques différentes de celles synthétisées par les mammifères ou de les produire en quantité non physiologiquement présente dans leurs organismes. On définit, selon l'invention par les termes « liquide biologique » tout liquide provenant d'un organisme vivant, en particulier d'un mammifère et plus particulièrement d'un humain et contenant de l'eau, au moins un type de protéine et/ou au moins un type de lipide et/ou au moins un type de sel minéral, présent sous forme d'ion, tel que, par exemple, sodium, le potassium, l'ion pouvant être métallique. On définit au sens de la présente invention un sel comme étant un sel minéral sous forme d'ion.

Les expérimentations décrites ci-après en référence à une infection fongique causée par *C. albicans* peuvent être reproduites avec des mammifères artificiellement infectés par un, deux ou plusieurs micro-organisme(s) fongique(s) pathogène(s) à étudier. Les expérimentations ci-dessous peuvent alors servir à déterminer le ou les composé(s) d'intérêt(s) apte à servir de marqueur de l'infection fongique invasive causée par le ou les micro-organisme(s) fongique(s) pathogène(s) à étudier, à caractériser ledit ou lesdits composés d'intérêt et à connaître sa relation avec l'infection.

### Partie Expérimentale

*Première étape :* Traitement des échantillons biologiques pour la détection des glycanes. Procédure appliquée aux sérums
   - Distribuer 300µL des échantillons à tester dans des microtubes de 1.5mL stériles.
   - Ajouter 100µL de solution de traitement (solution acide EDTA) dans
   - chaque tube.
   - Homogénéiser au « vortex ».
   - Placer les microtubes fermés hermétiquement avec des cavaliers de verrouillage pendant 6 mn à 120°C dans un bloc chauffant.
   - Retirer les tubes et les centrifuger 10mn à 10 000g.
   - Le surnageant (150µl) est transféré dans un microtube de 1.5ml stérile.
      Les traitements ultérieurs pour détection de glycanes par spectrométrie de masse sont effectués sur ce surnageant. Ce traitement libère/dissocie les mannanes et les galactomannanes des complexes protéiques coagulés dans le culot ; il n'altère en rien le dosage colorimétrique de glucane (kit Fungitell®) pour lequel il donne des résultats concordants avec le même échantillon non traité. Par contre ce traitement permet d'éliminer les interférences liées aux excès de protéines, de triglycérides, de bilirubine et/ou d'hémoglobine.
***Seconde étape* :** Traitement des surnageants pour purifier et concentrer les oligosaccharides préalablement à la spectrométrie de masse.
   100 µL de surnageant sont déposés sur une cartouche de Solid Phase Extraction (SPE) (référence SPE-C18: C18 Sep-Pak cartridge (Waters)) (1 volume) (colonne avec un garnissage hydrophobe), qui a été préalablement conditionnée par 5 volumes d'une solution d'acétonitrile (ANC) et d'eau (75 :25, vol/vol) et lavée par 10 volumes d'eau. Après pénétration du volume de surnageant recueilli dans la cartouche, celle-ci est rincée par deux volumes d'eau qui sont collectés. L'éluât de la colonne de C18 est déposé sur une cartouche SPE contenant un mélange en en poids égal de charbon actif commercial et de Célite ® (volume équivalent) préalablement conditionnée par 5 volumes d'une solution ANC/H2O (75 :25, vol/vol) puis rincée par 10 volumes d'eau. Après pénétration du volume de surnageant dans la cartouche, celle-ci est rincée par vingt volumes d'eau. L'éluât est jeté. La colonne est rincée par deux volumes d'une solution ANC/H2O (25 :75, vol/vol). Les cent premiers µL sont jetés. Les 300 µL suivants sont collectés. L'éluât (ACN/H2O) est séché.
***Troisième étape*:** Analyse des oligosaccharides par spectrométrie de masse. Le spectromètre de masse utilisé est un spectromètre MALDI-TOF Voyager Elite DE-STR (Perspective Biosystems Framingham, MA). L'éluât sec est solubilisé dans l'eau. 1 µL de solution est additionné de 1 µL d'une solution d'acide di-hydro benzoïque (10 mg/mL dans ACN/H2O 50 :50). 1 µL de la solution est déposé sur une plaque MALDI-TOF (plaque Applied Biosystems, acier inoxydable, 96 dépôts, cerclée) et cristallisé à 50°C. L'analyse MALDI-TOF est réalisée en mode positif-réflectron selon des paramètres d'acquisition optimisés pour l'observation d'oligosaccharides neutres présentant un rapport m/z inférieur à 1000. Après l'acquisition des donnée, la présence d'oligoglucanes dans le mélange est établie par l'observation des adduits [M+Na]+ générés. Les masses (M) d'intérêt pour le diagnostic sont calculées selon la formule M Hexₙ=180+[162]₍ₙ₋₁₎+23. La quantification relative des espèces moléculaires s'effectue en calculant le rapport des signaux M Hexₙ, soit sur un signal ubiquitaire endogène, soit sur un étalon externe introduit dans l'échantillon lors de la première phase de la seconde étape. A ce jour, le signal qui présente un intérêt pour le diagnostic est M Hex₂ = 365. Les exemples d'analyses présentés dans la suite du texte sont basés sur le calcul du rapport des signaux 365/361.

### Analyse de sérums humains

La méthode de l'invention a été appliquée à 12 sérums de patients hospitalisés au CHRU de Lille et sélectionnés sur les critères suivants :
i) avoir développé au cours dé leur hospitalisation une candidose systémique prouvée par au moins une hémoculture à *C*. *albicans*
ii) avoir bénéficié de la prescription d'un test de détection de la glucanémie ou de la mannanémie qui se soit révélé positif isolément ou conjointement et ayant orienté ou confirmé le diagnostic sur un sérum prélevé dans la semaine qui précède ou dans les 2 semaines qui suivent le prélèvement pour hémoculture s'étant révélé positif et
iii) la disposition d'un volume minimal de 1 mL de sérum résiduel stocké à - 20°C durant un an pour des raisons légales

Le tableau I ci-dessous récapitule les résultats obtenus par utilisation de kits Platelia® Candida antigen et Fongitell® sur les douze sérums de patients répondant aux critères précités et donc atteints de MFI.

**Tableau I**

| Echantillon de sérum | mannanes tel que détecté par le Platelia Candida Antigen® (pg/mL) | glucanes tels que détectés par le test Fungitell® (pg/mL) |
|---|---|---|
| G3 | | 11 |
| G4 | 0 | 0 |
| G5 | | 62 |
| G6 | | 11 |
| G10 | >500 | 126 |
| G12 | 0 | 178 |
| G15 | 0 | 0 |
| G21 | 216 | 232 |
| G25 | 610 | 43 |
| G32 | 0 | >500 |
| G42 | | 1272 |
| G49 | >500 | 388 |

La méthode selon l'invention a été appliquée à des échantillons des sérums précités. Les résultats sont visibles sur les Fig. 1a à 1d et sur les Fig. 2a à 2c.

Comme représenté sur les Fig. 1a à 1d, l'analyse des plaques MALDI réalisées à partir des sérums G25, G32, G42 et G49 du tableau I soumis au procédé de l'invention montre, d'une part, qu'il existe un signal de grande intensité correspondant à une masse m/z =361 quel que soit le sérum. Ce signal correspond à un composant inconnu du sérum et sert d'étalon d'intensité du signal. D'autre part, un signal apparaît sur le spectre de tous les sérums précités à une position correspondant à un rapport m/z=365. Ce signal correspond à un dimère d'hexose. Ce signal étant proche du signal ubiquitaire précité, ce dernier peut effectivement être utilisé comme signal étalon.

Par ailleurs, les spectres de plaques MALDI préparées selon le procédé de l'invention à partir de sérums de patients sains (résultats non représentés) ne comportent pas le signal correspondant à un rapport m/z=365 mais comportent bien le signal ubiquitaire correspondant à un rapport m/z=361. Dès lors, il est possible d'en déduire que dimère d'hexose qui est en quantité suffisante pour être détectable par spectrométrie de masse de type MALDI-TOF provient, directement ou non, du pathogène *Candida albicans.*

Les Fig. 2a à 2c montrent les résultats obtenus avec les kits commerciaux et les résultats obtenus par utilisation du procédé selon l'invention.

Le tableau II ci-dessous regroupe les résultats quantitatifs obtenus pour les échantillons de sérums en mettant en oeuvre la méthode de l'invention.

La méthode selon l'invention peut être considérée comme équivalente à la détection des mannanes présents dans l'échantillon de sérum ainsi qu'à celle des glucanes. En d'autres termes, le signal correspondant au dimère d'hexose précité (m/z=365) peut être considéré comme étant au moins caractéristique de la présence de mannanes et de glucanes dans l'échantillon de sérum concerné. De plus, on remarquera que pour les patients présentant à la fois une glucanémie et une mannanémie, le rapport de la hauteur du pic correspondant à m/z=365 sur la hauteur du pic correspondant à m/z=361 est relativement corrélé à la quantité de mannanes et de glucanes détectés. Pour le sérum G4, négatif avec les deux tests commerciaux, les résultats sont négatifs selon le procédé de l'invention (aucun signal à m/z=365). Par contre pour les patients G5, G6, G15 pour lesquels il n'a pas été possible de détecter des mannanes et pour lesquels les taux de glucanes détectés étaient très faibles ou nuls, un signal clair correspondant à un rapport m/z = 365 est identifiable.

**Tableau II**

| Echantillon de sérum | (hauteur du pic à m/z=365) /(hauteur du pic à m/z= 361x100 |
|---|---|
| G3 | 25 |
| G4 | 0 |
| G5 | 15 |
| G6 | 10 |
| G10 | 11 |
| G12 | 30 |
| G15 | 5 |
| G21 | 35 |
| G25 | 10 |
| G32 | 33 |
| G42 | 63 |
| G49 | 20 |

Le procédé de l'invention peut donc se substituer à la fois au Platelia® Candida Antigen et au Fungitell® en détectant des produits dont la présence est concomitante à la positivité de ces tests. De plus, le procédé selon l'invention permet de détecter un signal correspondant à m/z=365 même dans les sérums qui, au moyen des kits commerciaux s'étaient avérés comme étant dépourvus de glucanes et de mannanes. Le procédé de l'invention s'avère être donc plus sensible et plus fiable que les kits commerciaux précités.

Les résultats présentés ont été acquis sur un appareil de MALDI-TOF/MS en mode d'analyse dit 'réflectron'. Le mode d'analyse 'linéaire' en MALDI-TOF/MS permet d'obtenir les mêmes résultats.

### Caractérisation du composé d'intérêt donnant un signal à m/z=365

Pour déterminer la structure du composé d'intérêt servant de marqueur pour le diagnostic d'une infection fongique invasive et correspondant au pic m/z=365, on a réalisé la per-méthylation du surnageant des sérums de patients atteints de candidose invasive. Les sérums des patients atteints de candidose invasive sont donc traités selon les étapes une et deux, définies précédemment. On effectue ensuite la per méthylation des sucres contenus dans l'éluât sec obtenu à l'issue de la seconde étape et solubilisé dans l'eau. La technique de per méthylation utilisée est connue de l'Homme du Métier et décrite notamment dans la publication suivante : « Ciucanu I, Kerek F. 1984. A simple and rapid method for the per méthylation of carbohydrates. Carbohydr Res. 131:209-217». On réalise ensuite des plaques MALDI conformément au protocole décrit dans la troisième étape précitée. Le signal [M+Na]⁺ obtenu pour le sucre per méthylé apparaît pour une valeur de m/z= 477 ce qui correspond bien à un disaccharide Hexose-Hexose per méthylé.

Les sérums des patients atteints de candidose invasive, traités selon les deux premières étapes précitées et ayant subi une perméthylation présentent un pic chromatographique (chromatographie en phase gazeuse détecteur à ionisation de flamme GC-FID : colonne capillaire apolaire Alltech ECONO-CAP EC-1 [30m x 0.25mm x 0.25um]. Run-time = 28.8 min avec un gradient de température allant de 180C a 330C) correspondant à un temps de rétention identique à celui du tréhalose (αGlc1-1αGlc) perméthylé. L'analyse GC/EI-MS (chromatographie en phase gazeuse couplée à un spectromètre de masse de type impact électronique. colonne SGE SolGel-1ms [30m x 0.25mm x 0.25um]. Conditions expérimentales identiques à celles de le GC/FID) des sérums des patients diagnostiqués pour une candidose invasive, traités selon les deux premières étapes précitées et ayant subi une per méthylation présentent un spectre de fragmentation EI-MS identique à celui du tréhalose (αGlc1-1αGlc) per méthylé. Sur cette base, il est conclu que le composé d'intérêt détecté par spectrométrie de masse de type MALDI-TOF et correspondant à un signal à m/z=365 est un disaccharide et plus particulièrement du tréhalose. Ce composé d'intérêt peut donc être utilisé comme marqueur pour le diagnostic d'une infection fongique invasive, plus particulière d'une infection invasive causée par *C*. *albicans.*

### Détermination de l'origine fongique du composé d'intérét et de sa relation avec une infection fongique invasive.

### Expériences in vitro

Des granulocytes neutrophiles de souris ont été collectés dans la cavité abdominale de souris ayant reçu une injection intrapéritonéale de thioglycollate, selon la méthode décrite par Pluskota et al.(J Immunol. 2008 Sep 1;181(5):3609-19.). Six heures après l'injection précitée, les souris ont été sacrifiées par inhalation de dioxyde de carbone. La cavité abdominale fut ouverte et les granulocytes furent collectés par lavage de la cavité abdominale avec 5 ml d'une solution de PBS (solution tampon de phosphate salin) glacée. Pour réduire la possible contamination par les macrophages, les cellules ont été cultivées dans des boîtes de Pétri contenant du tissu de culture recouvert de sérum de veau foetal dans un incubateur à 37°C sous une atmosphère contenant 5% de dioxyde de carbone, pendant une heure. Les granulocytes non adhérents ont été collectés à partir du surnageant, centrifugées pendant 10 minutes à 2,250 g), comptés avec un hématimètre puis re-suspendues dans du Hank's Balanced Salt Solution (HBSS, commercialisé par la société Invitrogen, France). Des granulocytes humains furent isolés des cellules sanguines de sang de volontaires sains avec une solution acide de citrate de dextrose (1 volume de citrate 145 mM et 7 vol de dextrose à 2%, pH=4,6).L'isolement a été réalisé par centrifugation avec un appareil du type Ficoll-Hypaque, suivie par une sédimentation des érythrocytes dans du dextrane et une lyse hypotonique des érythrocytes résiduels.

### Evaluation de la destruction de C. albicans par les granulocytes.

Des levures (10⁶ unités) de la souche SC5314 de *C*. *albicans* ont été suspendues dans 0.25 ml de solution contenant 0.1 M HEPES (N-2-hydroxyethylpiperazine-N-2'-ethanesulfonic acid), pH 7.8, maintenues sous agitation à 1640 tours/min) et mélangées avec 3 X10⁶ granulocytes humains avec un ratio de 1:3 ratio. Le mélange granulocytes-levures a été incubé à 37°C sous une faible agitation pendant 1 à 2 heures. Toutes les 30 minutes, un volume aliquote à 300 µL du mélange est prélevé et utilisé pour détecter la présence ou non du composé d'intérêt correspondant à m/z=365. La détection du composé d'intérêt précité a été mise en oeuvre par spectrométrie de masse du type MALDI-TOF dans les mêmes conditions que précité.

Qu'il s'agisse des granulocytes humains ou murins, le signal correspondant au composé d'intérêt m/z=365 apparaît après environ deux heures ce qui prouve que le composé d'intérêt est probablement issu de l'interaction entre *C*. *albicans* et les granulocytes qui sont les cellules de la première ligne de défense des mammifères envers les pathogènes fongiques.

### Expériences in vivo chez la souris

Animaux : Des souris femelles C57BL/6 âgées de 6 à 8 semaines ont été utilisées pour toutes les expérimentations décrites dans la présente demande. Toutes les souris ont été fournies par les laboratoires Charles River Laboratories (France). Toutes les expérimentations animales ont été menées selon les protocoles approuvés par comité d'éthique en expérimentation animale du Centre Régional Hospitalier Universitaire de Lille et selon la directive européenne (86/609/CEE) relative à la protection des animaux utilisés à des fins expérimentales ou à d'autres fins scientifiques.

Quatre groupes de 10 souris chacun ont été créés. Tous les groupes de souris sont élevés 14 jours à partir de j=1 qui est le jour où débute l'expérimentation. A j=14, toutes les souris sont sacrifiées par dislocation des vertèbres cervicales. Leur sang est recueilli par ponction cardiaque. Les échantillons de sérum ont été réalisés à partir de ces prélèvements sanguins et stockés à -20°C avant leur utilisation pour la détection du composé d'intérêt correspondant à m/z=365. Le groupe 0 est le groupe témoin c'est-à-dire qu'il est composé de 10 souris ne recevant aucun traitement. La souris n'est naturellement (normalement) pas colonisée par *C. albicans.*

Le groupe 1 est le groupe des souris chez lesquelles on a induit une inflammation intestinale par ajout de 1.5% de DSS (sulfate de sodium de dextrane, MW 36-50 kDa; MP Biomedicals, LLC, Germany) dans l'eau de boisson donnée du jour j=1 à j=14. Ce groupe n'est pas colonisé par *C*. *albicans.*

Les souris du groupe 2 reçoivent par voie orale, à j=1, 200 mL de (PBS) contenant 10⁷ unités de *C. albicans.* Une inflammation intestinale est également induite chez les souris du groupe 2 par ajout de 1.5% de DSS (sulfate de sodium de dextrane, MW 36-50 kDa; MP Biomedicals, LLC, Germany) dans l'eau de boisson donnée du jour j=1 à j=14. L'administration de DSS qui provoque une inflammation intestinale rend la souris permissive à l'établissement de grandes quantités de levures dans son tube digestif. Ce modèle mime les patients hospitalisés et fortement colonisés par les Candida. Il s'agit de patients soumis à des radio ou chimiothérapies et qui présentent une altération des parois intestinales.

Les 10 souris du groupe 3 reçoivent à j=1 une injection intraveineuse dans la veine de la queue de 10⁴ unités de *C. albicans* de la souche SC5314 contenues dans 0.1 ml de solution saline stérile. Ce groupe mime une candidémie massive induisant une candidose invasive, d'issue fatale chez la souris. Ce modèle correspond à une candidémie (infection fongique invasive) chez le patient hospitalisé dont l'issue est très généralement fatale en l'absence de traitement. La présence ou non de mannanes dans les sérums des quatre groupes de souris a été testée avec le kit Platelia ® Candida antigen. Le tableau III ci-dessous regroupe les valeurs moyennes des dosages des mannanes présents dans les sérums de chacun des groupes 0 à 3 précités.

**Tableau III**

| Groupe de souris | Valeur moyenne de la concentration en mannanes contenus dans le sérum (pg/mL) |
|---|---|
| 0 | <1000 |
| 1 | 2800 |
| 2 | 4200 |
| 3 | 8400 |

Les résultats du Tableau III prouvent que le test Platélia ® Candida antigen s'avère être positif également dans le cas de souris colonisées mais qui ne sont pas atteintes de candidémie comme c'est le cas des souris du groupe 3.

On a ensuite détecté la présence ou non du composé d'intérêt correspondant à m/z=365 dans chacun des sérums de souris de chaque groupe. Le même protocole expérimental que précité en référence aux sérums de patients a été utilisé. Aucun signal à m/z=365 correspondant au composé d'intérêt n'a pu être détecté dans les sérums des souris des groupes 0, 1 et 2. Seul un signal à m/z=365 a pu être détecté par spectrométrie de masse de type MALDI-TOF dans chacun des sérums préalablement traités selon les deux premières étapes de la méthode de l'invention, des souris du groupe 3. Il en résulte que la méthode de l'invention permet de diagnostiquer de manière fiable une infection fongique invasive et de différencier une infection fongique invasive d'une colonisation massive du tractus digestif ce qui n'est pas le cas des kits commerciaux précités.

## Revendications

1. Méthode de diagnostic *in vitro* d'une infection fongique invasive causée par un micro-organisme fongique pathogène, **caractérisée en ce que** :
- on fournit un échantillon d'un liquide biologique provenant d'un mammifère, ledit liquide biologique contenant notamment, des protéines et/ou des lipides et/ou des sels et/ou des polysaccharides et/ou des oligosaccharides et/ou des monosaccharides susceptibles de former des complexes avec lesdites protéines et/ou lipides et/ou sels ;
- on traite ledit échantillon de liquide biologique de manière à extraire lesdits polysaccharides et/ou oligosaccharides et/ou monosaccharides ;
- on détermine par spectrométrie de masse de type MALDI-TOF la présence ou non, parmi lesdits les polysaccharides, oligosaccharides et/ou monosaccharides extraits, d'au moins un composé d'intérêt donné provenant dudit micro-organisme fongique et choisi parmi les polysaccharides, les oligosaccharides et les monosaccharides ;
- on en déduit que si ledit composé d'intérêt donné est présent dans ledit échantillon, ledit mammifère est atteint d'une infection fongique invasive.

2. Méthode selon la revendication 1, **caractérisée en ce que** pour extraire lesdits polysaccharides et/ou oligosaccharides et/ou monosaccharides dudit échantillon biologique :
- on dissocie lesdits complexes contenus dans ledit échantillon de liquide biologique par précipitation/coagulation de la majorité desdites protéines et/ou desdits lipides ;
- on centrifuge ledit échantillon et on sépare le surnageant de la fraction solide ;
- on récupère le surnageant ;
- on sépare lesdits polysaccharides et/ou oligosaccharides et/ou monosaccharides contenus dans le surnageant, des éventuelles protéines résiduelles et des éventuels lipides résiduels, notamment, par chromatographie de phase inverse et on sépare lesdits polysaccharides et/ou oligosaccharides et/ou monosaccharides desdits sels, en particulier, en réalisant une chromatographie d'absorption.

3. Méthode selon la revendication 2, **caractérisée en ce que** pour dissocier lesdits complexes, on ajoute un agent complexant, notamment une base, en particulier de l'EDTA et on chauffe ledit mélange obtenu, jusqu'à ébullition et notamment à une température comprise entre 100°C et 140°C et en particulier, sensiblement égale à 120°C.

4. Méthode selon l'une des revendications 2 et 3, **caractérisée en ce que** l'on effectue d'abord la chromatographie en phase inverse puis la chromatographie d'absorption.

5. Méthode selon l'une des revendications 3 et 4, **caractérisée en ce que** l'on utilise une colonne comportant une phase solide hydrophobe pour la chromatographie de phase inverse et une colonne contenant du charbon actif pour la chromatographie d'absorption.

6. Méthode selon l'une quelconque des revendications 1 à 5, **caractérisée en ce que** ledit composé d'intérêt présente un rapport m/z inférieur à 1000.

7. Méthode selon l'une quelconque des revendications précédentes, **caractérisée en ce qu'**on ajoute au moins une enzyme susceptible d'agir avec lesdits complexes et/ou on effectue un traitement chimique afin de couper les chaînes desdits polysaccharides et/ou oligosaccharides provenant de la dissociation desdits complexes, moyennant quoi, on augmente potentiellement la quantité dudit composé d'intérêt.

8. Méthode selon l'une quelconque des revendications précédentes, **caractérisée en ce que** ledit composé d'intérêt est choisi parmi les monomères et oligomères suivants : [N-acétyl-glucosamine β-(1,4) N-acétyl-glucosamine]n ou n est un entier supérieur ou égal à 1 et inférieur ou égal à 10, les oses, les oligosaccharides comprenant de 2 à 10 oses, notamment, les oligomères d'hexose, notamment, les dimères d'hexose, les glucanes, notamment les glucanes du type [glucose β-(1,3) glucose]ₙ, [glucose β-(1,6) glucose]ₙ où n étant un entier supérieur ou égal à 1 et inférieur ou égal à 10; les mannanes, notamment les mannanes du type [mannose α-(1,2) mannose]ₙ, [mannose α-(1,3) mannose]n, [mannose α-(1,6) mannose]ₙ, [mannose β-(1,2) mannose]ₙ où n est un entier supérieur ou égal à 1 et inférieur ou égal à 10, les galactomannanes, les galactanes, les arabinogalactanes et les glucuronoxylomannanes.

9. Méthode selon la revendication 8, **caractérisée en ce que** ledit composé d'intérêt est un composé produit par ledit micro-organisme fongique pathogène sous l'action du système immunitaire dudit mammifère et notamment le tréhalose.

10. Méthode selon l'une quelconque des revendications précédentes, **caractérisée en ce que** ledit micro-organisme fongique pathogène est choisi parmi *Candida spp., en particulier Candida albicans, Aspergillus spp., Fusarium spp., Trichosporon spp., Saccharomyces cerevisiae, Acremonium spp., Scedosporium spp., Coccidioides immitis, Histoplasma capsulatum, Sporothrix schenckii* et *Pneumocystis jirovecii* et les micro-organismes fongiques pathogènes produisant du tréhalose sous l'action du système immunitaire dudit mammifère.

11. Méthode selon l'une quelconque des revendications précédentes, **caractérisée en ce qu'**on ajoute audit échantillon une quantité connue d'au moins un composé étalon, moyennant quoi, en comparant l'intensité du signal obtenu pour ledit composé d'intérêt donné avec le signal obtenu pour ledit composé étalon, on peut déterminer la quantité dudit composé d'intérêt donné contenu dans ledit échantillon.

12. Méthode selon l'une quelconque des précédentes, **caractérisée en ce que** l'on détermine le rapport de l'intensité du signal correspondant au composé d'intérêt donné sur l'intensité d'un signal endogène ubiquitaire, ledit signal endogène étant préalablement identifié et éventuellement spécifique du type de liquide biologique dont provient l'échantillon, moyennant quoi on peut quantifier ledit composé d'intérêt donné contenu dans ledit échantillon.

13. Méthode selon l'une quelconque des revendications précédentes, **caractérisée en ce que** ledit liquide biologique est choisi parmi le sang veineux et artériel, le sérum, les liquides des muqueuses génitales, les liquides des voies aériennes, notamment, le liquide provenant de lavage broncho-alvéolaire, d'aspiration bronchique, d'aspiration trachéale, d' expectoration, les crachats, les liquides ayant servi à collecter des fragments de peaux et des phanères, les liquides d'épanchement, les liquides des cavités closes, notamment, le liquide céphalo-rachidien, les liquides du système digestif ou urinaire et les liquides obtenus à partir de broyat de fragment de biopsie.

14. Kit permettant la mise en oeuvre du procédé selon l'une quelconque des revendications précédentes, **caractérisé en ce qu'**il comprend :
- au moins un premier support comportant une phase solide hydrophobe permettant la mise en oeuvre d'une chromatographie en phase inverse ;
- au moins un deuxième récipient comportant une ouverture d'entrée, une ouverture de sortie et contenant un garnissage de charbon actif solide, disposé entre ladite ouverture d'entrée et ladite ouverture de sortie,
**en ce que** ledit support comportant une phase solide hydrophobe permettant la mise en oeuvre d'une chromatographie en phase inverse et ledit récipient contenant ledit garnissage de charbon actif sont choisis, indépendamment l'un de l'autre, parmi les éléments suivants : une plaque, un puits, une colonne, un tube et une plaque comportant une pluralité de puits ;
**en ce qu'**il comporte en outre, une plaque MALDI,
**en ce que** ledit garnissage est préparé en mélangeant une quantité égale de charbon actif et de terre de diatomée, et **en ce que** ladite phase solide hydrophobe se présente de préférence sous forme divisée et comporte un matériau magnétique.

15. Kit selon la revendication 14 , **caractérisé en ce qu'**il comprend une quantité donnée d'un composé étalon choisi parmi les monosaccharides, les disaccharides et les tri saccharides, éventuellement marqués par un isotope radioactif, et notamment le tréhalose deutéré et/ou en outre, une quantité donnée d'une enzyme apte à réagir avec les complexes formés par les polysaccarides et/ou les oligosaccharides et/ou monosaccharides contenus dans un liquide biologique.

## Patentansprüche

1. Verfahren zur in vitro-Diagnose einer invasiven Pilzinfektion, die durch einen pathogenen Pilzmikroorganismus verursacht wird, **dadurch gekennzeichnet, dass**:
- eine Probe einer biologischen Flüssigkeit bereitgestellt wird, die von einem Säuger stammt, welche biologische Flüssigkeit insbesondere Proteine und/oder Lipide und/oder Salze und/oder Polysaccharide und/oder Oligosaccharide und/oder Monosaccharide enthält, die geeignet sind, Komplexe mit den Proteinen und/oder Lipiden und/oder Salzen zu bilden;
- die Probe der biologischen Flüssigkeit behandelt wird, um die Polysaccharide und/oder Oligosaccharide und/oder Monosaccharide zu extrahieren;
- durch Massenspektrometrie vom Typ MALDI-TOF das Vorliegen oder nicht, unter den extrahierten Polysacchariden, Oligosacchariden und/oder Monosacchariden, mindestens einer Verbindung von gegebenem Interesse bestimmt wird, die von dem Pilzmikroorganismus stammt und aus den Polysacchariden, den Oligosacchariden und den Monosacchariden ausgewählt wird;
- daraus abgeleitet wird, dass, wenn die Verbindung von gegebenem Interesse in der Probe vorliegt, der Säuger an einer invasiven Pilzinfektion leidet.

2. Verfahren nach Anspruch 1, **dadurch gekennzeichnet, dass** zur Extraktion der Polysaccharide und/oder Oligosaccharide und/oder Monosaccharide aus der biologischen Probe:
- die Komplexe, die in der Probe der biologischen Flüssigkeit enthalten sind, durch Ausfällung/Koagulation der Mehrheit der Proteine und/oder der Lipide dissoziiert werden;
- die Probe zentrifugiert wird und der Überstand vom Feststoffanteil getrennt wird;
- der Überstand gewonnen wird;
- die Polysaccharide und/oder Oligosaccharide und/oder Monosaccharide, die im Überstand enthalten sind, von gegebenenfalls vorliegenden Restproteinen und gegebenenfalls vorliegenden Restlipiden getrennt werden, insbesondere durch Umkehrphasenchromatographie, und die Polysaccharide und/oder Oligosaccharide und/oder Monosaccharide von den Salzen getrennt werden, insbesondere unter Durchführung einer Absorptionschromatographie.

3. Verfahren nach Anspruch 2, **dadurch gekennzeichnet, dass**, um die Komplexe zu dissoziieren, ein Komplexbildner, insbesondere eine Base, besonders von EDTA, zugesetzt wird und die erhaltene Mischung bis zum Sieden und insbesondere auf eine Temperatur erhitzt wird, die zwischen 100 °C und 140 °C liegt und besonders im Wesentlichen gleich 120 °C ist.

4. Verfahren nach einem der Ansprüche 2 und 3, **dadurch gekennzeichnet, dass** zuerst die Umkehrphasenchromatographie, dann die Absorptionschromatographie durchgeführt wird.

5. Verfahren nach einem der Ansprüche 3 und 4, **dadurch gekennzeichnet, dass** eine Säule, die eine hydrophobe Festphase umfasst, für die Umkehrphasenchromatographie verwendet wird, und eine Säule, die Aktivkohle enthält, für die Absorptionschromatographie.

6. Verfahren nach einem der Ansprüche 1 bis 5, **dadurch gekennzeichnet, dass** die Verbindung von Interesse ein m/z-Verhältnis von weniger als 1000 aufweist.

7. Verfahren nach einem der vorhergehenden Ansprüche, **dadurch gekennzeichnet, dass** mindestens ein Enzym zugesetzt wird, das geeignet ist, mit den Komplexen zu agieren, und/oder eine chemische Behandlung durchgeführt wird, um die Ketten der Polysaccharide und/oder Oligosaccharide zu zerschneiden, die von der Dissoziation der Komplexe stammen, wodurch die Menge der Verbindung von Interesse potenziell erhöht wird.

8. Verfahren nach einem der vorhergehenden Ansprüche, **dadurch gekennzeichnet, dass** die Verbindung von Interesse aus den folgenden Monomeren und Oligomeren ausgewählt wird: [N-Acetylglucosamin-β-(1,4)-N-acetylglucosamin]ₙ, wobei n eine ganze Zahl größer oder gleich 1 und kleiner oder gleich 10 ist; Osen, Oligosacchariden, die 2 bis 10 Osen umfassen, insbesondere Oligomeren von Hexose, insbesondere Dimeren von Hexose, Glucanen, insbesondere Glucanen vom Typ [Glucose-β-(1,3)-glucose]ₙ,[Glucose-β-(1,6)-glucose]ₙ, wobei n eine ganze Zahl größer oder gleich 1 und kleiner oder gleich 10 ist; Mannanen, insbesondere Mannanen vom Typ [Mannose-α-(1,2)-mannose]ₙ, [Mannose-α-(1,3)-mannose]ₙ, [Mannose-α-(1,6)-mannose]ₙ, [Mannose-β-(1,2)-mannose]ₙ, wobei n eine ganze Zahl größer oder gleich 1 und kleiner oder gleich 10 ist, Galactomannanen, Galactanen, Arabinogalactanen und Glucuronoxylomannanen.

9. Verfahren nach Anspruch 8, **dadurch gekennzeichnet, dass** die Verbindung von Interesse eine Verbindung, die von dem pathogenen Pilzmikroorganismus unter Einwirkung des Immunsystems des Säugers erzeugt wird, und insbesondere Trehalose ist.

10. Verfahren nach einem der vorhergehenden Ansprüche, **dadurch gekennzeichnet, dass** der pathogene Pilzmikroorganismus ausgewählt wird aus Candida spp., besonders Candida albicans, Aspergillus spp., Fusarium spp., Trichosporon spp., Saccharomyces cerevisiae, Acremonium spp., Scedosporium spp., Coccidioides immitis, Histoplasma capsulatum, Sporothrix schenckii und Pneumocystis jirovecii, und die pathogenen Pilzmikroorganismen Trehalose unter Einwirkung des Immunsystems des Säugers erzeugen.

11. Verfahren nach einem der vorhergehenden Ansprüche, **dadurch gekennzeichnet, dass** der Probe eine bekannte Menge mindestens einer Standardverbindung zugesetzt wird, wobei durch den Vergleich der Intensität des Signals, das für die Verbindung von gegebenem Interesse erhalten wird, mit dem Signal, das für die Standardverbindung erhalten wird, die Menge der Verbindung von gegebenem Interesse bestimmt werden kann, die in der Probe enthalten ist.

12. Verfahren nach einem der vorhergehenden Ansprüche, **dadurch gekennzeichnet, dass** das Verhältnis der Intensität des Signals, das der Verbindung von gegebenem Interesse entspricht, zur Intensität eines endogenen ubiquitären Signals bestimmt wird, welches endogene Signal vorher identifiziert wird und gegebenenfalls für den Typ der biologischen Flüssigkeit spezifisch ist, von der die Probe stammt, wodurch die Verbindung von gegebenem Interesse quantifiziert werden kann, die in der Probe enthalten ist.

13. Verfahren nach einem der vorhergehenden Ansprüche, **dadurch gekennzeichnet, dass** die biologische Flüssigkeit ausgewählt wird aus venösem und arteriellem Blut, Serum, Flüssigkeiten der Genitalschleimhäute, Flüssigkeiten der Atemwege, wobei die Flüssigkeit insbesondere von einer bronchoalveolären Lavage, von einer bronchialen Absaugung, von einer trachealen Absaugung, von einer Expektoration stammt, Speichel, Flüssigkeiten, die dazu dienten, Fragmente von Haut und Hautanhangsgebilden zu sammeln, Effusionsflüssigkeiten, Flüssigkeiten aus geschlossenen Hohlräumen, insbesondere Cerebrospinalflüssigkeit, Flüssigkeiten des Verdauungs- oder Harnsystems und Flüssigkeiten, die aus zerkleinerten Biopsiefragmenten erhalten wurden.

14. Kit, welcher die Durchführung des Verfahrens nach einem der vorhergehenden Ansprüche ermöglicht, **dadurch gekennzeichnet, dass** dieser umfasst:
- mindestens einen ersten Träger, der eine hydrophobe Festphase umfasst, welche die Durchführung einer Umkehrphasenchromatographie ermöglicht;
- mindestens einen zweiten Behälter, der eine Eingangsöffnung, eine Ausgangsöffnung umfasst und eine Füllung mit fester Aktivkohle enthält, die zwischen der Eingangsöffnung und der Ausgangsöffnung angeordnet ist,
dass der Träger, der eine hydrophobe Festphase umfasst, welche die Durchführung einer Umkehrphasenchromatographie ermöglicht, und der Behälter, der die Füllung mit Aktivkohle enthält, unabhängig voneinander aus den folgenden Elementen ausgewählt werden: einer Platte, einer Senke, einer Säule, einem Rohr und einer Platte, die mehrere Senken umfasst;
dass er außerdem eine MALDI-Platte umfasst,
dass die Füllung hergestellt wird, indem eine gleiche Menge an Aktivkohle und an Diatomeenerde gemischt werden, und dass die hydrophobe Festphase vorzugsweise in geteilter Form vorliegt und ein magnetisches Material umfasst.

15. Kit nach Anspruch 14, **dadurch gekennzeichnet, dass** dieser umfasst: eine gegebene Menge einer Probenverbindung, die ausgewählt ist aus Monosacchariden, Disacchariden und Trisacchariden, gegebenenfalls markiert mit einem radioaktiven Isotop, und insbesondere deuterierter Trehalose, und/oder außerdem eine gegebene Menge eines Enzyms, das geeignet ist, mit den Komplexen zu reagieren, welche von den Polysacchariden und/oder Oligosacchariden und/oder Monosacchariden gebildet werden, die in einer biologischen Flüssigkeit enthalten sind.

## Claims

1. Method of *in vitro* diagnosis of an invasive fungal infection caused by a pathogenic fungal microorganism, **characterised in that**:
- a sample of biological fluid is provided, taken from a mammal, with said biological fluid notably containing proteins and/or lipids and/or salts, and/or polysaccharides and/or oligosaccharides and/or monosaccharides susceptible to form complexes with said proteins and/or lipids and/or salts;
- said biological fluid sample is treated so as to extract said polysaccharides and/or oligosaccharides and/or monosaccharides;
- using MALDI-TOF mass spectrometry, it is determined whether or not there is a presence, among said polysaccharides and/or oligosaccharides and/or monosaccharides extracted, of at least one given compound of interest deriving from said fungal microorganism and chosen from among polysaccharides, oligosaccharides and monosaccharides;
- from this it is inferred that if the said given compound of interest is present in the said sample, the said mammal is suffering from an invasive fungal infection.

2. Method according to claim 1, **characterised in that** to extract said polysaccharides and/or oligosaccharides and/or monosaccharides of said biological sample:
- said complexes contained in said sample of biological fluid are separated by precipitation/coagulation of the majority of proteins and/or lipids;
- said sample is centrifuged and the supernatant of the solid fraction is separated;
- the supernatant is recovered;
- said polysaccharides and/or oligosaccharides and/or monosaccharides contained in the supernatant are separated from any residual proteins and any residual lipids, notably by reversed-phase chromatography, and said polysaccharides and/or oligosaccharides and/or monosaccharides are separated from said salts, particularly by conducting adsorption chromatography.

3. Method according to claim 2, **characterised in that** to separate said complexes, a complexing agent is added, notably a base, in particular EDTA, and said obtained mixture is heated to boiling point, and notably to a temperature between 100°C and 140°C, and specifically roughly equal to 120°C.

4. Method according to one of claims 2 and 3, **characterised in that** firstly the reversed-phase chromatography is conducted, then adsorption chromatography is conducted.

5. Method according to one of claims 3 and 4, **characterised in that** a column comprising a hydrophobic solid phase is used for the reversed-phase chromatography, and a column containing activated carbon is used for the adsorption chromatography.

6. Method according to any of claims 1 to 5, **characterised in that** said compound of interest presents an m/z ratio of under 1000.

7. Method according to any one of the previous claims, **characterised in that** at least one enzyme able to act with said complexes is added, and/or chemical treatment is undertaken in order to cut the chains of said polysaccharides and/or oligosaccharides deriving from the separation of said complexes, whereby the quantity of said compound of interest is potentially increased.

8. Method according to any one of the previous claims, **characterised in that** said compound of interest is chosen from among the following monomers and oligomers: [N-acetyl-glucosamine β-(1,4) N-acetyl-glucosamine]n, where n is an integer greater than or equal to 1 and less than or equal to 10, the oses, the oligosaccharides comprising from 2 to 10 oses, notably the hexose oligomers, notably the hexose dimers, the glucans, notably the glucans of the type [glucose β-(1,3) glucose]ₙ, [glucose-β-(1,6) glucose]ₙ, where n is an integer greater than or equal to 1 and less than or equal to 10; the mannans, notably mannans of the type [mannose α-(1,2) mannose]ₙ, [mannose α-(1,3) mannose]ₙ, [mannose α-(1,6) mannose]ₙ, [mannose β-(1,2) mannose]ₙ, where n is an integer greater than or equal to 1 and less than or equal to 10, galactomannans, galactans, arabinogalactans and glucuronoxylomannans.

9. Method according to claim 8, **characterised in that** said compound of interest is a compound produced by said pathogenic fungal microorganism under the action of the immune system of the said mammal and notably trehalose.

10. Method according to any one of the previous claims, **characterised in that** said pathogenic fungal microorganism is chosen among *Candida spp., particularly Candida albicans, Aspergillus spp., Fusarium spp., Trichosporon spp., Saccharomyces cerevisiae, Acremonium spp., Scedosporium spp., Coccidioides immitis, Histoplasma capsulatum, Sporothrix schenckii* and *Pneumocystis jirovecii* and the pathogenic fungal microorganism producing trehalose under the action of the immune system of said mammal.

11. Method according to any one of the previous claims, **characterised in that** a known quantity of at least one standard compound is added to said sample, whereby, it is possible to determine the quantity of said given compound of interest contained in the said sample by comparison with the intensity of the signal related to said standard compound.

12. Method according to any one of the previous claims, **characterised in that** the ratio of the intensity of the signal corresponding to said compound of interest over the intensity of a ubiquitous endogenic signal is determined, with said endogenic signal being identified in advance, and possibly specific to the type of biological fluid of the sample, whereby it is possible to quantify said given compound of interest in said sample.

13. Method according to any one of the previous claims, **characterised in that** said biological fluid is chosen among venous and arterial blood, serum, genital mucous membrane fluids, airway fluid, notably the fluid from bronchoalveolar lavage, bronchial aspiration, tracheal aspiration, expectoration, sputum, fluids having been used to collect fragments of skin and skin appendages, cerebrospinal fluid, fluids of the digestive or urinary system, and fluids obtained from grinding a biopsy fragment.

14. Kit enabling the implementation of the process according to any of the previous claims, **characterised in that** it comprises:
- at least a first support comprising a hydrophobic solid phase enabling the implementation of reversed-phase chromatography;
- at least a second container comprising an entry opening, an exit opening, and containing a solid activated carbon lining, arranged between the said entry opening and the said exit opening,
**in that** said support comprising a hydrophobic solid phase enabling the implementation of reversed-phase chromatography and said container containing said activated carbon lining are chosen independently of each other, among the following elements: a plate, a well, a column, a tube and a plate comprising multiple wells;
**in that** it furthermore comprises a MALDI plate,
**in that** the said lining is prepared by mixing an equal quantity of activated carbon and diatomaceous earth, and **in that** said hydrophobic solid phase preferably is powder-like and comprises a magnetic material.

15. Kit according to claim 14, **characterised in that** it comprises a given quantity of a standard compound chosen among monosaccharides, disaccharides and trisaccharides, possibly marked by a radioactive isotope, and notably deuterated trehalose, and/or furthermore a given quantity of an enzyme able to react with the complexes formed by the polysaccharides and/or oligosaccharides and/or monosaccharides contained in a biological fluid.
